(19) Europäisches Patentamt
European Patent Office
Office européen des brevets

(11) Publication number: **0 476 339 A1**

(12) # EUROPEAN PATENT APPLICATION

(21) Application number: **91113988.9**

(51) Int. Cl.[5]: **C07C 319/02**, C07C 321/02

(22) Date of filing: **21.08.91**

(30) Priority: **19.09.90 US 585350**

(43) Date of publication of application:
**25.03.92 Bulletin 92/13**

(84) Designated Contracting States:
**AT BE CH DE DK ES FR GB GR IT LI LU NL**

(71) Applicant: **ATOCHEM NORTH AMERICA, INC.**
**Three Parkway**
**Philadelphia, Pennsylvania 19102(US)**

(72) Inventor: **Chen, Mabel Mei-Mei**
**80 Roberts Road**
**Newtown Square, Pennsylvania 19073(US)**

(74) Representative: **Kraus, Walter, Dr. et al**
**Patentanwälte Kraus, Weisert & Partner**
**Thomas-Wimmer-Ring 15**
**W-8000 München 22(DE)**

(54) **Process of manufacturing arylalkyl and alkyl mercaptans.**

(57) A process is disclosed for the manufacture of arylalkyl mercaptans, alkyl mercaptans or mixtures thereof by reacting the corresponding halide of said mercaptans with an alkali metal hydrogen sulfide in an aqueous, organic solvent-free solution, at an inert, low elevated pressure atmosphere of hydrogen sulfide, at an elevated temperature not exceeding 115 °C, and in the presence of a phase transfer catalyst.

EP 0 476 339 A1

## BACKGROUND OF THE INVENTION

This invention is a process for the preparation of arylalkyl and alkyl mercaptans by reacting an arylalkyl or alkyl halide with an alkali hydrogen sulfide.

## THE PRIOR ART

The preparation of a mercaptan from its corresponding halide and an alkali hydrogen sulfide is well known, having been disclosed, for example, in U.S. 2,147,400. More recently, U.S. Patent No. 4,740,623 has disclosed the preparation of benzyl mercaptan by reacting benzyl halide with alkali hydrogen sulfide in dilute aqueous solution at about 50 ° C until the reaction is 90% complete and then at 80 ° C, under a hydrogen sulfide atmosphere and in the absence of catalyst. A deficiency of this method is that it requires a large reaction vessel since a relatively dilute concentration of aqueous alkaline hydrogen sulfide, 5-30%, preferably 15%, is critical to the beneficial use of the process. With the employment of a large reaction vessel, low unit productivity and the generation of large amounts of waste are experienced.

Japanese patent publication 62/294652, published in December, 1987, has disclosed methods of preparing benzyl mercaptan derivatives in which a benzyl halide derivative is reacted with alkali hydrosulfide in either 1) a blended solvent of water and water-insoluble organic solvent, or 2) a water-soluble organic solvent. In the case of method 1), a phase transfer catalyst is employed. In the case of methods 1) and 2), an acid catalyst is preferably employed. The main drawback of these methods is the added cost of using an organic solvent, including the material cost, increased energy cost for solvent recovery, or cost of solvent disposal.

## STATEMENT OF THE INVENTION

This invention is a process for preparing a $C_7$ - $C_{22}$ arylalkyl or $C_1$ - $C_{12}$ alkyl mercaptan which comprises reacting a liquid $C_7$-$C_{22}$ arylalkyl halide or a $C_1$-$C_{12}$ alkyl halide with an alkali metal hydrogen sulfide in an aqueous, organic solvent-free solution having a sulfide concentration of from about 25 to about 40 weight percent, in an inert, low elevated pressure atmosphere comprising hydrogen sulfide, at an elevated temperature not exceeding about 115 ° C, and in the presence of an effective amount of a phase transfer catalyst.

## DETAILED DESCRIPTION OF THE INVENTION

The process described herein relates to the manufacture of arylalkyl mercaptans, alkyl mercaptans and mixtures thereof by reacting the corresponding halide of said mercaptan with an alkali hydrogen sulfide under prescribed conditions.

The alkyl group of the halide reactant has from 1 to 12 and preferably 2 to 8 carbon atoms and is either straight or branched chained. Examples of such alkyl groups are methyl, ethyl, propyl, isopropyl, butyl, isobutyl, octyl, and dodecyl. The aryl moiety of the arylalkyl group is either a phenyl or naphthyl radical. The halide ion of this compound is preferably the chloride ion because of its availability and price. However, the bromide, fluoride or iodide ion may be used in place of the chloride.

The alkali metal component of the alkali metal hydrogen sulfide is preferably sodium but potassium or lithium may also be used. The sulfide is dissolved in water to provide an aqueous solution thereof at a sulfide concentration of from about 25 to about 40, preferably about 30 to 35 weight %. Alternatively, the sulfide is generated in situ by passing gaseous hydrogen sulfide through aqueous sodium hydroxide until the desired concentration is reached.

The reaction of this invention is carried out under an atmosphere of hydrogen sulfide at low pressure generally between about 10 and about 60 psig, although the preferred range of pressure is from about 15 to about 35 psig. The use of hydrogen sulfide at low pressure in this process serves two purposes. In the aqueous solution of alkali metal hydrogen sulfide, an equilibrium reaction is established represented by the equation:

$$2\ MSH \rightleftharpoons M_2S + H_2S$$

(where M is an alkali metal cation). The presence of hydrogen sulfide drives the reaction to the left to foster the predominance of alkali metal hydrogen sulfide from which the nucleophile SH is generated. Secondly, the displacement of air in the reaction vessel with hydrogen sulfide reduces the chance of conversion of the mercaptan into a disulfide by oxidation. Thus, sufficient hydrogen sulfide is present in the reaction procedure to favorably influence the equilibrium reaction of the alkali metal hydrogen sulfide solution although another gas or gases, inert to the reaction, may be present with the hydrogen sulfide to thereby both prevent oxidation of the product mercaptan and drive the reaction toward the formation of alkali metal hydrogen sulfide.

A critical aspect of this invention is the use of a catalyst of the phase transfer type to hasten the reaction process. A "phase transfer" catalyst is a substance which may promote a reaction between reagents which are mutually immiscible. The phase

transfer catalyst suitable to enhance the liquid-liquid phase reaction between the aqueous alkali metal hydrosulfide and the organic aralkyl or alkyl halide includes members of the phase transfer catalyst group in general. Examples are, the quaternary salts including the ammonium, phosphonium and arsonium salts of the halides (chloride, bromide, iodide), sulfate and bisulfate, preferably containing at least 13 carbon atoms. Specific examples include tricaprylylmethyl ammonium chloride, tetrabutyl ammonium chloride, trioctylmethyl ammonium chloride, benzyltriethyl ammonium chloride, tetrabutyl phosphonium chloride, tetrabutyl ammonium bisulfate, hexadecyltriethyl ammonium bromide, tetrabutyl ammonium bromide, hexadecyltributyl phosphonium chloride, tetrabutyl phosphonium bromide, benzyltributyl arsonium chloride, and the like; Crown ethers or macrocylic polyethers, e.g., 12-crown-4 (1,4,7,10-tetraoxacyclodecane), 15-crown-5 (1,4,7,10,13-pentaoxacyclopentadecane), 18-crown-6 (1,4,7,10,13,16-hexaoxacyclooctadecane), dibenzo - 18 crown-6, dicyclohexano - 18 crown-6; Cryptands or macrotricylic diaminopolyethers, e.g, [2.2.1]-cryptate, [2.2.2]-cryptate, polyalkylene glycols and their ethers, e.g. compounds having the formula

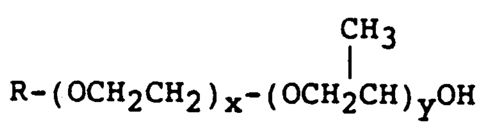

where R is independently hydrogen, alkyl, aryl, or alkaryl wherein the alkyl moieties have from 1 to 24 carbon atoms, preferably 1-12 carbons and x and y are independently values between 0 to 24 provided that either x or y equals at least 1, e.g, tetraethylene glycol, polyethylene glycol 400 - 1000, polyethylene glycol methyl ether 350-9000, and the like; an amine-based polyalkylene glycol such as tris(3,6-dioxaheptyl)amine and the like. Mixture of two or more phase transfer catalysts may also be beneficially used in the process of this invention to speed the reaction.

The phase transfer catalyst is used in an effective amount to hasten the reaction, preferably from about 0.1 to 20 mole% based on the weight of the starting arylalkyl or alkyl halide. Most preferably, the total catalyst amount used is from about 0.5 to 10 mole% based on the weight such halide.

The temperature at which the reaction is conducted is elevated but not exceeding about 115°C. Preferably the temperature range is from about 50°C up to 115°C, more preferably from about 70 to about 105°C to maintain a desirable rate of reaction.

## Examples

To demonstrate the slow nature of the reaction in the absence of a phase transfer catalyst the following experiment was performed.

An aqueous solution of sodium hydrogen sulfide (NaSH), having a concentration of 33.5 weight percent NaSH, was charged into a reaction vessel under partial vacuum (200 mmHg). The solution used weighed 34.1g and contained 11.4g (0.204 mole) of pure NaSH and a remainder of water. Hydrogen sulfide was introduced into the reaction vessel, under pressure, to reach 30 psig. When the temperature of the reaction mixture reached 80°C with external heating, 23.3g (0.2 mole) benzyl chloride was pumped into the reaction vessel. After 4 hours of heating at 80°C and agitation, the organic layer, after separation, was assayed by gas chromatography (GC) and found to contain 39 weight % of benzyl mercaptan.

The following examples serve to illustrate the process of this invention.

## Example 1

The procedure of the above experiment was repeated except with the addition of 0.8g (0.002 mole) of polyethylene glycol 400 (a phase transfer catalyst) to the aqueous NaSH at the start. At the end of the 4 hour reaction time, the organic layer, after separation, was assayed by GC and found to contain 83 weight % of benzyl mercaptan. High purity benzyl mercaptan (99.6 weight %) was obtained after vacuum distillation, b.p. 74°C/10 mn.

## Example 2

The procedure of Example 1 was repeated except that polyethylene glycol methyl ether 350 was used in the amount of 0.7g (0.002 mole) to replace polyethylene glycol 400 as the phase transfer catalyst. The separated organic layer was assayed by GC and found to contain 88 weight % of benzyl mercaptan.

## Example 3

The procedure of Example 1 was repeated except that tris(3,6-dioxaheptyl)amine (TDA-1) in the amount of 0.65g (0.002 mole) was used to replace the polyethylene glycol 400 as the phase transfer catalyst. The separated organic layer was assayed by GC and found to contain 70 weight % of benzyl mercaptan.

## Example 4

The procedure of Example 1 was repeated

except that tricaprylylmethyl ammonium chloride (Aliquat® 336) in the amount of 0.81g (0.002 mole) was used to replace the polyethylene glycol 400 as the phase transfer catalyst. The separated organic layer was assayed by GC and found to contain 65 weight % of benzyl mercaptan.

## Claims

1. A process for preparing a $C_7$-$C_{22}$ arylalkyl or $C_1$-$C_{12}$ alkyl mercaptan which comprises reacting a liquid $C_7$-$C_{22}$ arylalkyl or $C_1$-$C_{12}$ alkyl halide with an alkali metal hydrogen sulfide in an aqueous, organic solvent-free solution having a sulfide concentration of from about 25 to about 40 weight percent, in a low elevated pressure atmosphere comprising hydrogen sulfide at an elevated reaction temperature not exceeding about 115 °C, and in the presence of an effective amount of a phase transfer catalyst.

2. The process of claim 1 wherein the halide reactant is an arylalkyl chloride and the alkali metal hydrogen sulfide is sodium hydrogen sulfide.

3. The process of claim 1 wherein said aqueous solution has a concentration of at least 30% by weight of alkali metal hydrogen sulfide.

4. The process of claim 1 wherein the low elevated pressure ranges from about 10 to about 60 psig.

5. The process of claim 1 wherein the elevated temperature ranges from about 50 to about 115 °C.

6. The process of claim 2 wherein the halide reactant is benzyl chloride.

7. The process of claim 1 wherein the phase transfer catalyst is an ammonium, phosphonium or arsonium quaternary salt.

8. The process of claim 7 wherein the salt is tricaprylylmethyl ammonium chloride.

9. The process of claim 1 wherein the phase transfer catalyst is a polyalkylene glycol or ether thereof having one or more alkylene radicals containing from 2 to 4 carbon atoms.

10. The process of claim 9 wherein the phase transfer catalyst is a polyethylene glycol having an average molecular weight of at least about 200.

11. The process of claim 9 wherein the phase transfer catalyst is a polyethylene glycol ether having an average molecular weight of at least about 300.

12. The process of claim 1 wherein the phase transfer catalyst is an amine-based polyalkylene glycol.

13. The process of claim 12 wherein the amine-based polyalkylene glycol is tris(3,6-dioxaheptyl)amine.

14. The process of claim 6 wherein said aqueous solution has a concentration of at least 30% by weight of sodium hydrogen sulfide, the low elevated pressure ranges from about 10 to about 60 psig, the elevated temperature ranges from about 50 to 115 °C, and the amount of phase transfer catalyst ranges from 0.1 to 20 mole % based on the starting weight of benzyl chloride.

15. The process of claim 14 wherein said pressure ranges from about 15 to about 35 psig, the temperature ranges from about 70 and about 105 °C, and the amount of said catalyst ranges from about 0.5 to about 10 mole %.

16. The process of claim 15 wherein said phase transfer catalyst is a polyalkyene glycol or ether thereof having one or more alkylene radicals containing from 2 to 4 carbon atoms.

17. The process of claim 16 wherein said catalyst is polyethylene glycol having an average molecular weight of at least 200.

18. The process of claim 16 wherein said catalyst is polyethylene glycol methyl ether having an average molecular weight of at least 300.

19. The process of claim 15 wherein said catalyst is tris(3,6-dioxaheptyl)amine.

20. The process of claim 15 wherein said catalyst is tricaprylylmethyl ammonium chloride.

European Patent Office

# EUROPEAN SEARCH REPORT

Application Number

EP 91113988.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | PATENT ABSTRACTS OF JAPAN, unexamined applications, C field, vol. 12, no. 191, June 3, 1988 THE PATENT OFFICE JAPANESE GOVERNMENT page 49 C 501 * Kokai-no. 62-294 652 (NISSAN CHEM IND) * -- | 1,2,5, 7 | C 07 C 319/02<br>C 07 C 321/02 |
| X | EP - A - 0 268 261 (PHILLIPS PETROLEUM) * Claims 1,2,4 * -- | 1,5,7 | |
| A | EP - A - 0 337 838 (SOCIETE NATIONALE ELF AQUITAINE) * Abstract * -- | 1,3,5-7 | |
| D,A | US - A - 4 740 623 (JAMES B. HEATHER) * Abstract * -- | 1-3,5 | TECHNICAL FIELDS SEARCHED (Int. Cl.5) |
| A | SOVIET INVENTIONS ILLUSTRATED, Ch section, week 83/29, August 31, 1983 DERWENT PUBLICATIONS LTD., London * SU-956 569 (MAZAEV) * -- | 1 | C 07 C 319/00<br>C 07 C 321/00 |
| A | SOVIET INVENTIONS ILLUSTRATED, Ch section, week E 06, March 24, 1982 DERWENT PUBLICATIONS LTD., London * SU-825 515 (MAZAEV) * -- | 1 | |
| A | US - A - 2 395 240 (WALTER V. WIRTH) * Claim 1 * | 1 | |

The present search report has been drawn up for all claims

| Place of search | Date of completion of the search | Examiner |
|---|---|---|
| VIENNA | 05-12-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

-2-
EP 91113988.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | -- | | |
| D,A | US - A - 2 147 400 (LEE H. CLARK et al.) * Claim 1 * ---- | 1 | |
| | | | TECHNICAL FIELDS SEARCHED (Int. Cl.5) |

The present search report has been drawn up for all claims

| Place of search | Date of completion of the search | Examiner |
|---|---|---|
| VIENNA | 05-12-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document